# EUROPEAN PATENT APPLICATION

(11) **EP 4 442 115 A1**
(43) Date of publication of application: **09.10.2024**
(21) Application number: 23908692.9
(22) Date of filing: 21.12.2023
(51) Int. Cl.: A01N 1/02, C12N 5/00

(54) **CELL TRANSPORTATION METHOD**

(30) Priority: 26.12.2022 CN 202211673559
(71) Applicant: Shenzen Junxing Biotechnology Co., Ltd., Shenzhen, Guangdong 518110 (CN)
(72) Inventor: DU, Mingchun, Shenzhen, Guangdong 518110 (CN); QU, Kangning, Shenzhen, Guangdong 518110 (CN)
(74) Representative: Gulde & Partner
(86) International application number: PCT/CN2023/140565
(87) International publication number: WO 2024/140406

(57) **Abstract**

The present invention relates to the field of biotechnology, specifically to a method for transporting cells. In the present invention, the cells can be mixed with a material and then directly transported at ambient temperature; or the cells can be mixed with a material, subject to three-dimensional culture, and then transported at ambient temperature. During the transportation, the cells can keep a high survival rate of up to 80% or more; and after the transportation, the cell/material mixture can be directly used, or the cells can be isolated for use.

## Description

### TECHNICAL FIELD

The present invention relates to biotechnology, specifically to a method for transporting cells.

### BACKGROUND

Cell therapy, as a safe and effective treatment means, has been widely used in clinical research of diseases such as immune diseases, cancers, and genetic diseases, which involves cell collection, culture expansion, gene modification, cell transfusion/transplantation treatment, and other parts. Since various parts may have different implementation locations or time, the cells used in therapy often need to be transported between different locations on time. How to efficiently solve the problems in the cell transportation has become one of make-or-break factors in the cell therapy.

At present, there are mainly two methods for transporting cells, that is, cryopreservation transportation or liquid-filled ambient-temperature transportation. Among them, the cryopreservation transportation is carried out by freezing and placing the cells in a special vessel filled with liquid nitrogen or dry ice. It has good preservation effect, but is complicated in procedure and unsuitable for long-term transportation, which is generally carried out by air transportation with high cost. The liquid-filled ambient-temperature transportation is carried out by filling a culture flask containing adherent cells with a cell culture liquor. It is simply and practical, but is prone to undergo surge of the culture solution during transportation to cell detachment which will seriously affect the cell viability.

Therefore, there is an urgent need for a method for transporting cells which is low-cost, simple and easy-to-do, and do not substantially affect the cell viability during the transportation.

### SUMMARY OF THE INVENTION

A technical problem to be solved by the present invention is to provide a method for transporting cells which is low-cost, simple and easy-to-do, and do not substantially affect the cell viability during the transportation.

A method for transporting cells of the present invention comprises sequentially the steps of:
(1) taking an aqueous biomaterial solution out of a refrigerator at 4°C, sucking up and mixing an appropriate amount of the aqueous biomaterial solution with the cells uniformly;
(2) placing the cell/material mixture obtained in step (1) at 37°C to form a hydrogel structure encapsulating the cells;
(3) placing the hydrogel structure obtained in step (2) in a cell culture solution for transportation; and
(4) placing the hydrogel structure transported in step (3) at 4°C to remove the material, and isolating the cells by centrifugation.

A method for transporting cells comprises sequentially the steps of:
(1) taking an aqueous biomaterial solution out of a refrigerator at 4°C, sucking up and mixing an appropriate amount of the aqueous biomaterial solution with the cells uniformly;
(2) placing the cell/material mixture obtained in step (1) at 37°C to form a hydrogel structure encapsulating the cells;
(3) placing the hydrogel structure obtained in step (2) into a cell culture solution and performing a three-dimensional cell culture in a cell incubator;
(4) placing the hydrogel structure cultured in step (3) in a cell culture solution for transportation; and
(5) placing the hydrogel structure transported in step (4) at 4°C to remove the material, and isolating the cells by centrifugation.

In the method for transporting cells of the present invention, in various methods, the biomaterial comprises at least one of poloxamer, collagen, peptide-based material, chitosan and its derivative, starch-based material, serum albumin, clupeine, polylysine, carbomer, poly(methacrylic acid), poly(N-isopropyl acrylamide), polyacrylamide, acrylic acid, gelatin and its derivative, hyaluronic acid, gellan gum, polyvinyl alcohol, fibrinogen, keratin, fibronectin, reduced keratin, cellulose-based material, alginate and its derivative, and xylan.

In the method for transporting cells of the present invention, in various methods, the biomaterial has a concentration ranging from 0.001 wt.% to 20 wt.%.

In the method for transporting cells of the present invention, in step (1) of various methods, the cells have a density ranging from 1 × 10⁵ to 1 × 10⁹ cells/mL in the aqueous biomaterial solution.

In the method for transporting cells of the present invention, in the methods, the three-dimensional cell culture is carried out for a period ranging from 3 to 30 days.

In the method for transporting cells of the present invention, in various methods, temperature is controlled in a range from 4°C to 37°C during cell transportation.

In the method for transporting cells of the present invention, in various methods, the cells have a survival rate of more than or equal to 70% but less than or equal to 100%; and preferably, in various methods, the cells have a survival rate of more than or equal to 80% but less than or equal to 95%.

In the method for transporting cells of the present invention, in various methods, after the cell transportation, the hydrogel structure containing the cells can be directly used without removal of the material.

Compared with the existing technology, the method for transporting cells of the present invention involves encapsulating the cells with a biomaterial under mild conditions to form a three-dimensional hydrogel structure, immersing the hydrogel in a cell culture solution for transportation, and removing the biomaterial in a gentle way to achieve the isolation of cells after the transportation. This method is simple and easy-to-do, has low cost, and does not substantially affect the cell viability during the transportation.

### BRIEF DESCRIPTION OF THE DRAWINGS

FIG. 1 is an image showing living/dead cell staining effect of the cells in the hydrogel structure before and after the transportation in Example 1 (wherein, FIG. 1A shows the sample before the transportation, and FIG. 1B shows the sample after the transportation); and
FIG. 2 is an image showing living/dead cell staining effect of the cells in the hydrogel structure before and after the transportation in Example 2 (wherein, FIG. 2A shows the sample before the transportation, and FIG. 2B shows the sample after the transportation).

### DETAILED DESCRIPTION

### EXAMPLE 1

This example is a method for transporting cells comprising sequentially the steps of:
(1) taking an aqueous biomaterial solution containing 1% poloxamer and 1% collagen out of a refrigerator at 4°C, sucking up and mixing 1 mL of the aqueous biomaterial solution with mouse aortic vascular smooth muscle cells uniformly to a cell density of 1 × 10⁶ cells/mL;
(2) placing the cell/material mixture obtained in step (1) at 37°C to form a hydrogel structure encapsulating the cells;
(3) placing the hydrogel structure obtained in step (2) in a DEME-high-glucose cell culture medium containing 10% fetal bovine serum for transportation at ambient temperature; and
(4) placing the hydrogel structure which had undergone 5-day transportation in step (3) at 4°C to remove the material, and isolating the cells by centrifugation, wherein the cellular survival rate was 90%.

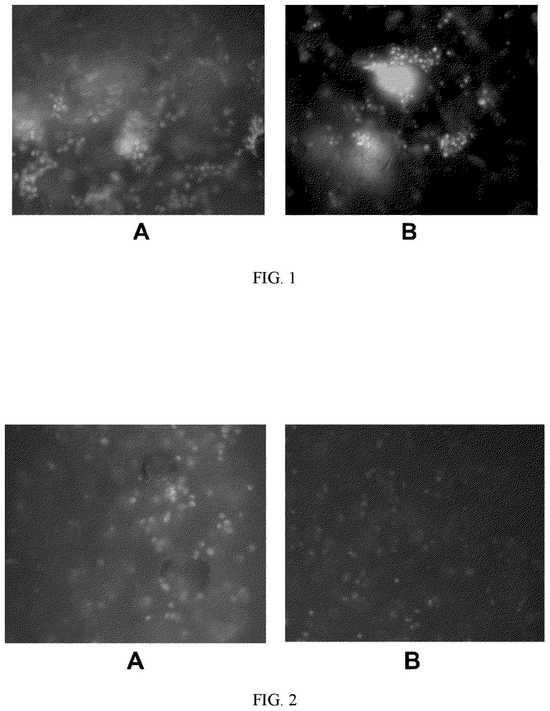

Before and after the cell transportation, the cells in the hydrogel structure were labeled with AO/PI double-staining reagent for marking living/dead cells; labeled with DAPI reagent for marking the nuclei; and observed under fluorescence microscopy for recording the cellular survival rate. Among them, Acridine Orange (briefly, AO) can cross intact cell membrane and be embedded into the nuclei of all cells (living and dead), showing a green fluorescence; Propidium iodide (briefly, PI) can only cross non-intact cell membrane (*i.e.,* cell membrane of dead cells) and be embedded into the nuclei of all dead cells, showing a red fluorescence; and DAPI can penetrate cell membrane and bind to a double-stranded DNA to show a blue fluorescence. The test results are shown in FIG. 1. As shown in the tests, the cells show a large number of green fluorescence signals in the hydrogel structure both before and after the transportation, and maintain a high survival rate.

### EXAMPLE 2

A method for transporting cells comprises sequentially the steps of:
(1) taking an aqueous biomaterial solution containing 5% polyacrylamide and 0.1% polylysine out of a refrigerator at 4°C, sucking up and mixing 2 mL of the aqueous biomaterial solution with human umbilical vein endothelial cells uniformly to a cell density of 1 × 10⁵ cells/mL;
(2) placing the cell/material mixture obtained in step (1) at 37°C to form a hydrogel structure encapsulating the cells;
(3) placing the hydrogel structure obtained in step (2) into a RPMI-1640 cell culture medium containing 10% fetal bovine serum, and performing a three-dimensional cell culture in a cell incubator for a culture time of 10 days;
(4) placing the hydrogel structure obtained in step (3) in a RPMI-1640 cell culture medium containing 10% fetal bovine serum for transportation at 37°C; and
(5) placing the hydrogel structure which had undergone 3-day transportation in step (4) at 4°C to remove the material, and isolating the cells by centrifugation, the cellular survival rate was 80%.

In the method for transporting cells of the present invention, the time of the three-dimensional cell culture in step (3) can be selected as any duration between 3 days and 30 days, in addition to the 10 days selected.

Example 2 differs from Example 1 in that the hydrogel structure obtained in step (2) was first placed in a cell culture solution and subject to a three-dimensional cell culture in an cell incubator before it was transported in a cell culture solution. Whether to perform a three-dimensional cell culture operation before the cell transportation depends mainly on two aspects of:
1. Whether the density of cells encapsulated in the hydrogel structure can meet the demand for cells during the post-transportation use. If the pre-transportation cell density can meet the actual demand for cells after the transportation, the cells may not be subject to a three-dimensional cell culture before the transportation, and can be directly used after the transportation; and if the pre-transportation cell density cannot meet the actual demand for cells after the transportation, the cells can be subject to a three-dimensional cell culture before the transportation to increase the cell density in the hydrogel structure by the proliferation behavior of the cells in the hydrogel structure so that the cells can be directly used after the transportation to meet the actual demand of cells.
2. Requirements for the cellular survival rate during the post-transportation use. If the requirement for cellular survival rate after the transportation is not high, e.g., a cell survival rate of 70% in the present invention, the cells may not be subject to a three-dimensional cell culture before the transportation, and the hydrogel structure mainly provides a three-dimensional physical support for the cell transportation and cannot provide a suitable micro-environment for cell growth, and would not substantially affect the survival rate of cells during the transportation. And if the requirement for cellular survival rate after the transportation is high, e.g., a cellular survival rate of 95% in the present invention, the cells can be subject to a three-dimensional culture so that they continue to proliferate during the culture, and secrete extracellular matrix to build a micro-environment suitable for the cell growth, which is more conducive to maintaining a high cellular survival rate during the transportation. Among them, the duration of the three-dimensional cell culture is related to the culture environments of different cells, growth status of cells, expected survival rates, post-transportation usage modes of cells, or similar factors. A suitable culture time can be selected in accordance with different practical requirements.

Before and after the cell transportation, the cells in the hydrogel structure were labelled with AO/PI double-staining reagent for marking living/dead cells; labelled with DAPI reagent for marking the nuclei; and observed under fluorescence microscopy for recording the cellular survival rate. Among them, Acridine Orange (briefly, AO) can cross intact cell membrane and be embedded into the nuclei of all cells (living and dead), showing a green fluorescence; Propidium iodide (briefly, PI) can only cross non-intact cell membrane (*i.e.,* cell membrane of dead cells) and be embedded into the nuclei of all dead cells, showing a red fluorescence; and DAPI can penetrate cell membrane and bind to a double-stranded DNA to show a blue fluorescence. The test results are shown in FIG. 2. As shown in the tests, the cells show a large number of green fluorescence signals in the hydrogel structure both before and after the transportation, and maintained a high survival rate.

The method for transporting cells of the present invention involves encapsulating the cells with a biomaterial under mild conditions to form a three-dimensional hydrogel structure, immersing the hydrogel in a cell culture solution for transportation at 4-34°C, and removing the biomaterial in a gentle way to achieve the isolation of cells after the transportation (or the hydrogel structure containing the cells can be directly used without removal of the material). The method is simple and easy-to-do, has low cost, and does not substantially affect the cell viability during the transportation. By testing, on the premise of ensuring the temperature to be controlled in a range of 4-37°C during the cell transportation, the cells transported by this method can have a cellular survival rate of more than or equal to 80% but less than or equal 95%.

The above-described examples are only for the purpose of illustrating the preferred embodiments of the present invention. Without departing from the design spirit of the present invention, various modifications and improvements made by those of ordinary skill in the art to the technical solutions of the present invention should fall within the scope of the present invention as defined by the appended claims.

### INDUSTRIAL APPLICABILITY

The method for transporting cells of the present invention is low-cost, simple, easy-to-do, does not substantially affect the cell viability during the transportation, and can be applied in cell therapy. It is the key to ensure successful implementation of cell therapy so that the cell therapy can be successfully implemented in the fields such as immune diseases, cancers, and genetic diseases.

## Claims

1. A method for transporting cells, **characterized by** comprising sequentially the steps of:
(1) taking an aqueous biomaterial solution out of a refrigerator at 4°C, sucking up and mixing an appropriate amount of the aqueous biomaterial solution with the cells uniformly;
(2) placing the cell/material mixture obtained in step (1) at 37°C to form a hydrogel structure encapsulating the cells;
(3) placing the hydrogel structure obtained in step (2) in a cell culture solution for transportation; and
(4) placing the hydrogel structure transported in step (3) at 4°C to remove the material, and isolating the cells by centrifugation.

2. A method for transporting cells, **characterized by** comprising sequentially the steps of:
(1) taking an aqueous biomaterial solution out of a refrigerator at 4°C, sucking up and mixing an appropriate amount of the aqueous biomaterial solution with the cells uniformly;
(2) placing the cell/material mixture obtained in step (1) at 37°C to form a hydrogel structure encapsulating the cells;
(3) placing the hydrogel structure obtained in step (2) into a cell culture solution and performing a three-dimensional cell culture in a cell incubator;
(4) placing the hydrogel structure cultured in step (3) in a cell culture solution for transportation; and
(5) placing the hydrogel structure transported in step (4) at 4°C to remove the material, and isolating the cells by centrifugation.

3. The method for transporting cells of claim 1 or 2, **characterized in that** in various methods, the biomaterial comprises at least one of poloxamer, collagen, peptide-based material, chitosan and its derivative, starch-based material, serum albumin, clupeine, polylysine, carbomer, poly(methacrylic acid), poly(N-isopropyl acrylamide), polyacrylamide, acrylic acid, gelatin and its derivative, hyaluronic acid, gellan gum, polyvinyl alcohol, fibrinogen, keratin, fibronectin, reduced keratin, cellulose-based material, alginate and its derivative, and xylan.

4. The method for transporting cells of claim 3, **characterized in that** in various methods, the biomaterial has a concentration ranging from 0.001 wt.% to 20 wt.%.

5. The method for transporting cells of claim 1 or 2, **characterized in that** in step (1) of various methods, the cells have a density ranging from 1 × 10⁵ to 1 × 10⁹ cells/mL in the aqueous biomaterial solution.

6. The method for transporting cells of claim 2, **characterized in that** in step (3), the three-dimensional cell culture is carried out for a period ranging from 3 to 30 days.

7. The method for transporting cells of claim 1 or 2, **characterized in that** in various methods, temperature is controlled in a range from 4°C to 37°C during cell transportation.
